# EUROPEAN PATENT SPECIFICATION

(11) **EP 2 687 607 B1**
(45) Date of publication and mention of the grant of the patent: **04.03.2015**
(21) Application number: 12005291.5
(22) Date of filing: 19.07.2012
(51) Int. Cl.: C12Q 1/68

(54) **MicroRNA profiles in the diagnosis of multiple sclerosis**
Mikro-RNA-Profile bei der Diagnose von multipler Sklerose
Profils de micro-ARN dans le diagnostic de la sclérose en plaques

(43) Date of publication of application: 22.01.2014
(73) Proprietor: St. Josef- und St. Elisabeth-Hospital GmbH, 44791 Bochum (DE); Medizinische Hochschule Hannover, 30625 Hannover (DE)
(72) Inventor: Thum, Thomas, 30659 Hannover (DE); Haghikia, Aiden, 44789 Bochum (DE); Haghikia, Arash, 30159 Hannover (DE); Gold, Ralf, 44799 Bochum (DE)
(74) Representative: Forstmeyer, Dietmar

(56) References cited:
- WO-A1-2011/003989
- WO-A2-2008/153692
- WO-A2-2011/163214
- MARTINELLI-BONESCHI F ET AL.: "MicroRNA and mRNAexpression profile screening in multiple sclerosis patients to unravel novel pathogenic steps and identify potential biomarkers", NEUROSCIENCE LETTERS, vol. 508, 2 February 2012 (2012-02-02), pages 4-8, XP002690297,
- OTAEGUI D ET AL: "Differential Micro RNA Expression in PBMC from Multiple Sclerosis Patients", PLOS ONE, vol. 4, no. 7, E6309, 1 July 2009 (2009-07-01), pages 1-9, XP002558932, PUBLIC LIBRARY OF SCIENCE, US ISSN: 1932-6203, DOI: 10.1371/JOURNAL.PONE.0006309 [retrieved on 2009-07-20]
- KELLER A ET AL.: "Multiple sclerosis: MicroRNA expression profiles accurately differentiate patients with relapsing-remitting disease from healthy controls", PLOS ONE, vol. 4, no. 10, E7440, October 2009 (2009-10), pages 1-7, XP002690298,
- HAGHIKIA A ET AL.: "Regulated microRNAs in the CSF of patients with multiple sclerosis", NEUROLOGY, vol. 79, 27 November 2012 (2012-11-27), pages 1-5, XP002690299,

## Description

### Field of the Invention

The invention provides a method of determining if a patient is afflicted with multiple sclerosis (MS) using microRNA (miRNA) profiles of specific miRNAs that are present in the cerebrospinal fluid (CSF). This method can also be used to discriminate different MS disease courses. The invention further comprises a kit for diagnosing or monitoring MS based upon the miRNA profiles according to the invention, and also relates to the use of said miRNA profiles in the diagnosis or monitoring of MS.

### Background of the Invention

miRNAs are small non-coding RNAs (17-24 nucleotides) that regulate gene expression by binding to partly complementary sequences in messenger RNA transcripts (mRNAs) thereby preventing the mRNAs from being translated into protein. Due to their function as regulators of gene expression they play a critical role in fundamental biological processes, including hematopoietic differentiation, cell cycle regulation, metabolism, cardiovascular biology, and immune function, and have been suggested to be involved in pathological processes, such as cancer, inflammatory diseases and neurological diseases, e.g. MS (Junker, Hohlfeld and Meinl, Nat Rev Neurol 2011; 7: 56-59). It has been found that the expression profile (or expression pattern) of miRNAs varies over time and between tissues/cells. These findings make miRNA a potential tool for diagnostics for various types of diseases, e.g. neurological diseases (see, e.g. WO 2008/153692) and lung cancer (see, e.g. WO 2010/139810). Interestingly, miRNAs are also detectable outside cells, e.g. in body fluids such as blood, plasma, serum or urine, and thus may serve as novel diagnostic/prognostic biomarkers (Widera et al., J Mol Cell Cardiol. 2011 Nov;51(5):872-5; Lorenzen et al., Clin J Am Soc Nephrol. 2011 Jul;6(7):1540-6).

Multiple sclerosis (MS), also known as "disseminated sclerosis" or "encephalomyelitis disseminata" is an autoreactive immune mediated, inflammatory, demyelinating disease of the central nervous system (CNS) with aspects of neurodegeneration over time. In MS, the myelin surrounding nerve cells is damaged or destroyed, impacting the ability of the nerves to conduct electrical impulses to and from the brain. These damaged areas are also known as "plaques" or "lesions". Neuronal tissue damaged within and aside the lesions will eventually lead to irreversible disease progression and disability.

MS has a prevalence that ranges between 2 and 150 per 100,000, is almost three times more common in women, and has a wide ranging age of onset, however, with a peak between the ages of 20 and 40years. MS patients can suffer almost any neurological symptom or sign, including visual problems (e.g. blurred or double vision, or red-green color distortion), problems in speech, muscle weakness, difficulties with coordination and balance, tremors, fatigue, loss of sensitivity, and cognitive impairments.

MS takes several forms, with new symptoms occurring either in discrete attacks (relapsing forms) or slowly accumulating over time (progressive forms). Between attacks, symptoms may be completely reversible. Neurological and neuro-radiological expert panels have created diagnostic criteria to ease and standardize the diagnostic process, differentiating between the following MS subtypes: relapsing-remitting multiple sclerosis (RRMS), secondary progressive multiple sclerosis (SPMS), primary progressive multiple sclerosis (PPMS). Overlapping subtypes occur regularly and may indicate transition phases, e.g. SPMS with super-imposed relapses.

RRMS that accounts for the beginning of more than 90% of all MS cases is characterized by clearly-defined, acute attacks (relapses), usually with full or partial recovery; disease progression can occur between attacks. SPMS is initially relapsing-remitting but then becomes continuously progressive at a variable rate, with or without occasional relapses along the way. Approximately 50% of RRMS eventually transition to SPMS. However, these numbers are vague and there are no reliable indicators for the transition phase. Primary progressive MS may be characterized by disease progression from the beginning with few or no periods of remission. Progressive-relapsing MS is characterized by disease progression from the beginning, but with clear, acute relapses along the way.

The putative role of miRNAs in MS emerges from miRNA profiling analyses in active MS lesions, where it was shown that upregulation of three distinct miRNAs - miR-34a, miR-155 and miR-326 - promote macrophage activity (Junker et al., Brain 2009; 132: 3342-3352). Findings from an animal model of MS also support the involvement of miRNAs - in particular miR-124 - in the pathophysiology of MS (Ponomarev et al., Nat Med 2011; 17: 64-70). Human MS studies showed altered miRNA expression patterns in whole blood samples, peripheral blood monocytes, lymphocyte subpopulations, and serum samples of MS patients (Junker, Hohlfeld and Meinl, loc. cit., Otaegui et al., PLoS ONE 2009; 4: e6309; WO 2011/163214).

However, the results of these studies are widespread and in some cases reveal conflicting conclusions. For instance miR-17 and miR-20a, which belong to the miR-17-92 cluster, were significantly down-regulated in all MS subtypes (Cox et al., PLoS One 2010; 5:e12132), whereas, miRNAs of the same cluster were up-regulated in another study (Lindberg et al., Eur J Immunol 2010; 40: 888-898). In a study analyzing miRNAs in CSF of CNS-lymphoma patients high stability of miRNAs has been demonstrated: neither exposure to RNase, nor repeated freeze-thaw cycles and long-term storage of CSF samples affected miRNA levels in the CSF (Baraniskin et al., Blood 2011; 117: 3140-3146).

The variable clinical presentation of MS and the lack of established diagnostic laboratory tests can lead to delays in the diagnosis of MS. Additionally, there are no clinically established laboratory tests available which allow to distinguish between different MS disease courses. As a result, a need remains to provide easily applicable methods to effectively diagnose MS, especially since early immunomodulatrly therapy is the only available way to prevent gross disease progression and disability.

It is, therefore, an aim of the present invention to provide a novel alternative method for a diagnosis of MS. Another object of the invention is to provide a method for determining the MS subtype and a kit for diagnosing MS. The kit and methods of the invention are preferably more robust, objective, definitive, and rapid as current diagnostic tools thereby improving the lives of the patients. An earlier and/or more precise identification of MS and the MS subtype, respectively, would allow the medical practitioner an earlier and a more effective treatment.

### Summary and Description of the Invention

The present invention was made in view of the prior art and the needs described above, and, therefore, the object of the present invention is to provide a novel alternative method for diagnosing MS.

More specifically, the inventors found that miR-181c_5p, miR-633 and miR-922 are differentially expressed in CSF samples of patients afflicted with MS as compared to relevant controls, e.g. other neurological diseases (OND).

The inventors showed that the expression level of miR-633 is up-regulated, while the expression level of miR-922 is down-regulated in CSF samples of patients afflicted with MS as compared to relevant controls (OND).

The inventors, moreover, showed that the expression level of miR-181c_5p is up-regulated in CSF samples of a patient afflicted with MS compared to relevant controls (OND).

Finally, the inventors showed that miR-181c_5p and miR-633 are differentially expressed in RRMS and SPMS thereby allowing to differentiate between MS disease courses. More specifically, the inventors showed that the expression level of miR-181c_5p and miR-633 is down-regulated in RRMS compared to SPMS.

These surprising and unexpected results for the first time allow a diagnostic and disease monitoring value to be conceived for CSF-based miR-181c_5p, miR-633 and miR-922 in the diagnosis of MS and in the differentiation of MS disease courses.

Accordingly, the invention provides a method of determining if a patient is afflicted with MS using miRNA profiles of specific miRNAs that are present in the cerebrospinal fluid (CSF). The method is based on the determination of specific miRNAs that have altered expression levels in disease state (MS) compared to relevant controls.

Other objects of the present invention are to provide a method for discriminating different disease courses of MS, to provide a kit for diagnosing or monitoring MS based upon the miRNA profiles according to the invention, and to use said miRNA profiles in the diagnosis of MS.

These objects are solved by the subject matter of the attached claims.

The aspects of the present invention will become apparent upon reference to the following detailed description.

The present invention is directed to a method of determining if a patient is afflicted with multiple sclerosis (MS), comprising determining in a cerebrospinal fluid (CSF) sample from the patient the expression profile of miR-633 and miR-922, wherein miR-633 is up-regulated (increased concentration) and miR-922 is down-regulated (lower concentration) in a patient who is afflicted with MS.

A sample from a patient as used herein means a test sample from a human subject suspected to be affected by a disease. The method may also involve obtaining a test sample of cerebrospinal fluid (CSF) from the subject. For example, cerebrospinal fluid can be obtained by lumbar puncture.

As used herein "determining the expression profile of miRNAs in a sample" means assaying a test sample, e.g. a CSF sample from a patient, *in vitro* to determine the concentration or amount of the miRNAs in the sample. Any convenient qualitative, semi-quantitative or, preferably, quantitative detection method for determining nucleic acids can be used to determine the concentration or amount of the miRNAs in the sample. A variety of methods for determining nucleic acids are well known to those of skill in the art, e.g. determination by nucleic acid hybridization and/or nucleic acid amplification. Exemplary methods to determine the concentration or amount of the miRNAs in the sample are provided below.

The concentration or amount of the miRNAs in the sample may be directly determined in the CSF test sample, that is, without an RNA extraction step. Alternatively, RNA may be extracted from the CSF sample prior to miRNA processing for detection. RNA may be purified using a variety of standard procedures as described, for example, in RNA Methodologies, A laboratory guide for isolation and characterization, 2nd edition; 1998, Robert E. Farrell, Jr., Ed., Academic Press. In addition, there are various processes as well as products commercially available for isolation of small molecular weight RNAs, including miRNeasy™ kit (Qiagen), MagMA™ kit (Life Technologies), Pure Link™ kit (Life Technologies), and mirVANA™ miRNA Isolation Kit (Ambion). For example, small molecular weight RNAs may be isolated by organic extraction followed by purification on a glass fiber filter. Alternative methods for isolating miRNAs include hybridization to magnetic beads.

The diagnosis or monitoring is based on comparing the patient's expression levels of the miRNAs in the sample with those obtained using relevant controls, e.g. internal standards, samples of CSF from subjects known to be free of the disease or known to suffer from the same or a different disease. In cases where the method is being used to monitor a patient with MS or to test for the recurrence or progression of MS, the "control" may be test results obtained from the same patient at an earlier time, i.e., the patient may be examined for changes in microRNA levels before and after a certain treatment.

It will be understood that it is not absolutely essential that an actual control sample be run at the same time that assays are being performed on a test sample. Once "normal," i.e., control, levels of the miRNAs (or of miRNA ratios) have been established, these levels can provide a basis for comparison without the need to rerun a new control sample with each assay.

The comparison between the test and control samples provides a basis for a conclusion as to whether a subject has MS (in cases where the method is being used diagnostically) or whether MS is progressing or regressing in response to therapy (in cases where the method is being used for monitoring). The expression levels of the analyzed miRNAs when statistically analyzed will have a threshold whereby expression levels of the individual miRNAs above or below the threshold are indicative for the presence or absence of MS or a MS subtype. Threshold miRNA levels for each of the analyzed miRNAs can be determined by any suitable algorithm. Such an algorithm may involve classifying a sample between MS and non-MS groups. For example, samples may be classified on the basis of threshold values as indicated, or based upon Mean and/or Median miRNA levels in MS patients versus a non-MS population (e.g., a cohort from the general population or a patient cohort with diseases other than MS). Various classification schemes are known for classifying samples between two or more groups, including Decision Trees, Logistic Regression, Principal Components Analysis, Naive Bayes model, Support Vector Machine model, and Nearest Neighbor model. In addition, the predictions from multiple models can be combined to generate an overall prediction.

The invention thereby allows a robust, rapid and effective diagnosis or monitoring of MS with a high sensitivity and specificity for the differentiation between MS-afflicted patients (e.g., relapsing-remitting MS patients) and non-MS afflicted patients. For instance, the method according to the invention distinguishes a MS-afflicted patient from a non-MS afflicted patient with a sensitivity of at least about 50%, 75%, 80%, 85%, or 88%. The specificity of the method for distinguishing a MS-afflicted patient from a non-MS afflicted patient may be at least about 50%, 60%, 69%, or greater. The sensitivity for the discrimination between MS subtypes, e.g. RRMS and SPMS, in the method according to the invention may be at least about 50%, 60%, 69%, or greater. The specificity of the method for discriminating between MS subtypes may be at least about 50%, 75%, 80%, 82%, or greater. In this respect, the method according to this aspect may lend additional or alternative predictive value over standard clinical methods of diagnosing MS, such as for example, absence or presence of lesions on an MRI, testing positive or negative for oligoclonal bands, or the absence or presence of other signs and symptoms of MS such as blurred vision, fatigue, and/or loss of balance.

The miRNA expression profile (miRNA signature, or miRNA concentration) is generated (determined) from (in) the CSF-samples using any of various methods known in the art for quantifying miRNA levels. Such methods include polymerase-based assays, such as Real-Time PCR (e.g., Taqman™), hybridization-based assays, for example using microarrays (e.g. miRNome microRNA Profilers QuantiMir Human PCR array (Biocat)), nucleic acid sequence based amplification (NASBA), flap endonuclease-based assays, as well as direct RNA capture with branched DNA (QuantiGene™), Hybrid Capture™ (Digene), or nCounter™ miRNA detection (nanostring). The assay format, in addition to determining the miRNA levels will also allow for the control of, inter alia, intrinsic signal intensity variation. Such controls may include, for example, controls for background signal intensity and/or sample processing, and/or hybridization efficiency, as well as other desirable controls for quantifying miRNA levels across samples (e.g., collectively referred to as "controls"). Many of the assay formats for amplifying and quantitating miRNA sequences, and thus for generating miRNA profiles are commercially available and/or have been described, e.g. in WO 2008/153692, WO 2010/139810, and WO 2011/163214, or references cited therein.

The specific CSF-based miRNAs that are tested for in the present invention include miR-181c_5p (sometimes also referred to as miR-181c), miR-633 and miR-922. The designations provided are standard in the art and are associated with specific sequences that can be found at the microRNA registry (http://microrna.sanger.ac.uk/sequences/).

In all cases, unless otherwise explicitly specified, they refer to human sequences, which may be indicated with the prefix "hsa" (Homo sapiens), i.e. hsa-miR-181c, hsa-miR-633 and hsa-miR-922, under the standard nomenclature system. Although the miRNAs tested for are indicated as RNA sequences, it will be understood that, when referring to hybridizations or other assays, corresponding DNA sequences can be used as well. For example, RNA sequences may be reverse transcribed and amplified using the polymerase chain reaction (PCR) in order to facilitate detection. In these cases, it will actually be DNA and not RNA that is directly quantitated. It will also be understood that the complement of the reverse transcribed DNA sequences can be analyzed instead of the sequence itself. In this context, the term "complement" refers to an oligonucleotide that has an exactly complementary sequence, i.e. for each adenine there is a thymine, etc. Although assays may be performed for the miRNAs individually, it is generally preferable to assay several miRNAs or to compare the ratio of two or more of the miRNAs.

The method of the invention can comprise differentiating between MS and an other neurological disease (OND), wherein miR-633 is up-regulated (increased concentration) and miR-922 is down-regulated (lower concentration) in the CSF from a MS patient compared to a patient with an OND.

The term "other neurological disease" or "OND" as used herein means a disease selected from the group: polyneuropathy, migraine, scotoma, schizophrenia, syncopes, leukoencephalopathy (unclear etiology), fracture 4th lumbal vertebra, normal pressure hydrocephalus, Ormond's disease, psychosomatic disorder, fracture 12th thoracic vertebra, spinal hemangioma, epileptic seizure, transient ischemic attack, spinocerebellar ataxia, multiple system atrophy, Parkinson's disease, Lewy body dementia, and aseptic meningitis.

Preferably, the method of the invention can further comprise determining the expression profile of miR-181c in the cerebrospinal fluid (CSF) sample from the patient.

Also preferred, the method of the invention can further comprise determining the level of one or more normalization control(s) in the sample. Preferably, the sample can be spiked with the normalization control(s).

Preferred according to the invention, the normalization control can be a non-endogenous RNA or miRNA, or a miRNA not expressed in the sample. For example, the normalization control may be one or more exogenously added RNA(s) or miRNA(s) that are not naturally present in the patient, e.g. an RNA or miRNA from an other organism, e.g. C. elegans (e.g. C. elegnas miR-39) or Arabidopsis (e.g. ath-miR-159a), and/or one or more human miRNAs not expressed in the CSF-sample undergoing analysis.

In the method of the invention, the miRNA profile (or miRNA concentration) is preferably determined by an amplification- and/or hybridization-based assay. The amplification- and/or hybridization-based assay can be quantitative miRNA real-time polymerase chain reaction (RT-PCR), e.g. TaqMan. The miRNA profile may also be determined by preparing cDNA, followed by RT-PCR.

As discussed above, the method of the invention can also be used to monitor a patient with MS or to test for the recurrence or progression of MS, i.e. discriminate between forms (subtypes) of MS based upon the differential expression of miR-181c and miR-633 in the CSF from MS patients with RRMS and patients with progressive forms of MS. In such cases where the method is being used to monitor a patient with MS or to test for the recurrence or progression of MS, the method can further comprise discriminating between relapsing remitting multiple sclerosis (RRMS) and a progressive form of MS, wherein miR-181c_5p and miR-633 are down-regulated (lower concentration) in RRMS as compared to the progressive form of MS. The progressive form of MS is selected from among secondary progressive multiple sclerosis (SPMS), primary progressive multiple sclerosis (PPMS), and progressive relapsing multiple sclerosis (PRMS). Preferred according to the invention, the progressive form of MS to be diagnosed or monitored is SPMS but may be extended to other progressive courses of disease.

The miRNA profile may be prepared with the use of a custom kit or array, e.g., to allow particularly for the profiling of the CSF-based miRNAs associated with MS. Accordingly, the present invention further provides a kit (or test) for diagnosing or monitoring MS based upon the miRNA profiles in the CSF as described herein.

The kit for diagnosing or monitoring MS of the invention may comprise means for determining the concentration (expression profile) of miR-633 and miR-922; or miR-181c_5p, miR-633 and miR-922; in a cerebrospinal fluid sample from a patient. The means for determining the concentration of miR-633, miR-922 and/or miR-181c_5p can be oligonucleotide probes specific for miR-633, miR-922 and/or miR-181c_5p; or miRNA-specific primers for reverse transcribing or amplifying each of miR-633, miR-922 and/or miR-181c_5p. For example, the means for determining the concentration of miR-633, miR-922 and/or miR-181c_5p may be TaqMan probes specific for each miRNA of the kit.

The design of oligonucleotide probes specific for miR-633, miR-922 and/or miR-181c_5p; or miRNA-specific primers for reverse transcribing or amplifying each of miR-633, miR-922 and/or miR-181c_5p to detect their expression levels (concentrations) in accordance with suitable assay formats is well known to those of skill in the art, and appropriate probes and/or primers can be commercially purchased.

Further, the kit may comprise an enzyme for cDNA preparation (e.g. , reverse transcriptase) and/or PCR amplification (e.g., Taq polymerase), and/or a reagent for detecting and/or quantifying miRNA. Additionally, the kit may further comprise include a reagent for miRNA isolation from samples. The kit can also comprise one or more normalization control(s). The normalization control(s) can, for example, be provided as one or more separate reagent(s) for spiking samples or reactions. Preferably, the normalization control(s) is/are selected from non-endogenous RNA or miRNA, or a miRNA not expressed in the sample.

The invention further provides the use of a miRNA expression profile of miR-633 and miR-922 (concentrations of the respective miRNAs) in a CSF sample from a patient for diagnosing or monitoring MS. The miRNA profile (concentration) that is used for diagnosing or monitoring MS may further comprise the expression profile (concentration) of miR-181c_5p. The miRNA expression profile is indicative of the presence of MS if miR-633 and/or miR-181c_5p is/are up-regulated (increased concentration), and miR-922 is down-regulated (lower concentration) in the CSF sample.

The miRNA expression profile of miR-633 and miR-922 (concentrations of the respective miRNAs) in a CSF sample from a patient can also be used to differentiate between MS and an other neurological disease (OND). The miRNA expression profile that can be used to differentiate between MS and an other neurological disease (OND) may further comprise the expression profile (concentration) of miR-181c_5p. The miRNA expression profile (concentrations of the respective miRNAs in the sample) that can be used to differentiate between MS and an other neurological disease (OND) is indicative of the presence of MS if miR-633 and/or miR-181c_5p is/are up-regulated (increased concentration), and miR-922 is down-regulated (lower concentration) in the CSF from a MS patient compared to a patient with an OND. The OND is preferably an OND as described herein above.

The miRNA expression profile of miR-181c_5p and miR-633 (concentrations of the respective miRNAs) in a CSF sample from a patient can also be used to discriminate between relapsing remitting multiple sclerosis (RRMS) and a progressive form of MS, wherein miR-181c_5p and miR-633 are down-regulated (lower concentration) in RRMS as compared to the progressive form of MS. The progressive form of MS is selected from among secondary progressive multiple sclerosis (SPMS), primary progressive multiple sclerosis (PPMS), and progressive relapsing multiple sclerosis (PRMS); preferably, the progressive form is SPMS.

It is especially preferred to combine the preferred embodiments of the present invention in any possible manner.

### Description of the Figures

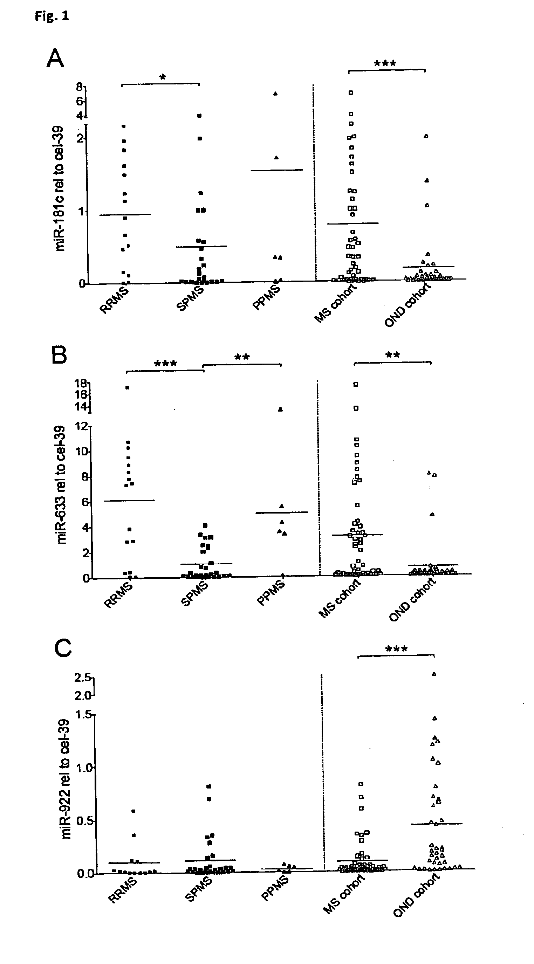

**Figure 1****.** Deregulated CSF based miRNAs in MS patients with relapsing remitting (RRMS), secondary progressive (SPMS) and primary progressive (PPMS) and the whole MS cohort as compared to patients with other neurological diseases (OND). The MS cohort includes RRMS, SPMS and PPMS separately shown on the left side of the diagrams. The y-axis depicts values normalized to spiked-in cel-miR-39 and expressed as (Starting Quantity [microRNA]/Starting Quantity [cel-miR-39]) for miR-181c (**A**), miR-633 (**B**) and miR-922 (**C**); black bars indicate mean values; *p<0.05, **p<0.01, ***p<0.001.

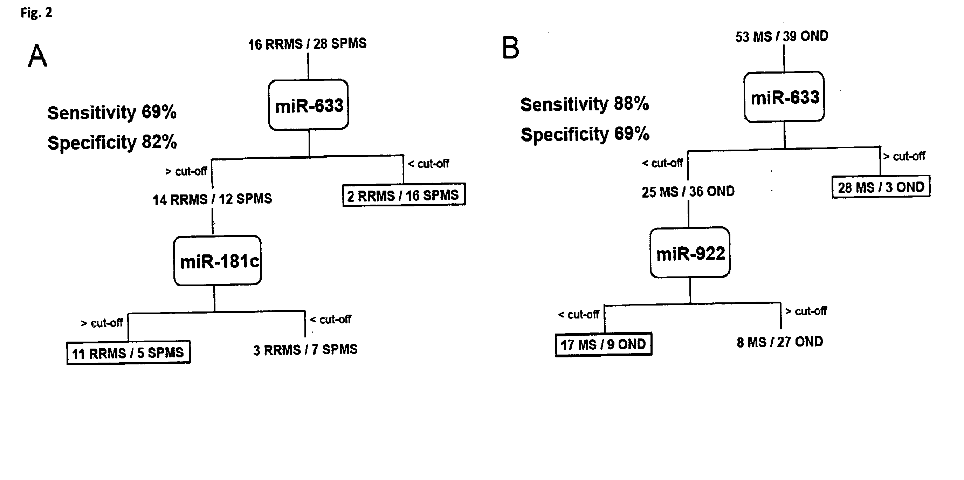

**Figure 2****.** Diagnostic tree. Combination of candidate miRNAs in a diagnostic tree resulted in considerable specificity and sensitivity values to differentiate (**A**) relapsing remitting (RRMS) from secondary progressive MS (SPMS) and (**B**) MS from patients with other neurological diseases (OND). Cut-offs used in the respective trees were: < 0.35 for miR-633 and > 0.47 for miR-181c to differentiate between RRMS and SPMS (A); > 0.73 for miR-633 and < 0.04 for miR-922 (B).

### Examples

### Patients and Methods

### Patients' characteristics

In accordance with the ethics committee of the Ruhr-University Bochum on CSF-sample collection, since February 2009 remaining CSF of samples obtained for routine diagnostic and therapeutic purposes after written informed consent, including MS and OND patients, were collected and stored at -80°C. For this study, we assessed CSF of n=53 MS patients with clinically well-defined disease courses and n=39 OND; hemorrhagic samples were excluded (details of patients' characteristics are summarized in **Tables 1** to **3** below).

**Table 1**

| **MS Patients** | **Age mean years (range)** | **Gender f : m** | **Disease duration mean years (range)** |
|---|---|---|---|
| RRMS (n=17) | 38.9 (18 - 52) | 3.25 : 1 | 5.5 (1 - 16) |
| SPMS (n=30) | 50.8 (35 - 63) | 2 : 1 | 15.6 (2 - 34) |
| PPMS (n=6) | 53.8 (48 - 61) | 1 : 1 | 14.7 (7 - 31) |

**Table 2**

| **OND patients** | **Age/ Gender** | **miR-181c_5p** | **miR-633** | **miR-922** |
|---|---|---|---|---|
| Polyneuropathy | 71/f | 0.017 | 0.183 | 0.781 |
| Migraine | 53/f | 0.036 | 0.182 | 0.425 |
| Scotoma | 26/m | 0.002 | 0.147 | 1.006 |
| Schizophrenia | 67/f | 0.006 | 0.076 | 0.067 |
| Syncopes | 48/m | 0.023 | 0.582 | 1.179 |
| Leukoencephalopathy (unclear etiology) | 48/f | 0.031 | 0.094 | 0.057 |
| Fracture 4th lumbal vertebra | 86/f | 0.347 | 0.412 | 1.422 |
| Normal pressure hydrocephalus | 62/m | 0.0 | 0.336 | 1.204 |
| Ormond's disease | 67/m | 0.078 | 0.71 | 2.481 |
| Psychosomatic disorder | 36/f | 0.047 | 0.155 | 0.007 |
| Fracture 12th thoracic vertebra | 68/f | 0.0 | 0.097 | 0.001 |
| Spinal hemangioma | 26/f | 0.0 | 0.1 | 0.007 |
| Epileptic seizure | 88/f | 0.001 | 0.143 | 0.0 |
| Transient ischemic attack | 58/m | 0.01 | 4.71 | 0.00094 |
| Spinocerebellar ataxia | 41/m | 0.198 | 0.347 | 0.66 |
| Multiple System Atrophy | 70/m | 0.002 | 0.175 | 0.465 |
| Parkinson's disease | 71/m | 0.0 | 0.087 | 0.603 |
| Parkinson's disease | 62/f | 0.213 | 0.339 | 1.242 |
| Lewy body dementia | 70/f | 0.016 | 0.247 | 0.155 |
| Aseptic meningitis | 37/m | 0.053 | 0.137 | 0.001 |

**Table 3**

| **Diagnosis Patients** | **Age/ Gender** | **miR-181c** | **miR-633** | **miR-922** |
|---|---|---|---|---|
| Dementia (unclear etiology) | 69/f | 0.0 | 0.053 | 0.131 |
| Motoneuron disease | 63/m | 0.001 | 0.048 | 0.019 |
| Hypesthesia lower limbs (MS excluded) | 44/m | 0.064 | 0.21 | 0.007 |
| Friedreich's ataxia | 30/f | 1.98 | 7.85 | 0.06437 |
| Huntington's disease | 48/f | 0.001 | 0.067 | 0.106 |
| Guillain-Barré syndrome | 53/m | 0.246 | 0.293 | 1.042 |
| Myelitis | 26/f | 0.014 | 0.318 | 0.689 |
| Neuromyelitis optica | 57/m | 0.067 | 0.379 | 0.448 |
| Acute demyelinating encephalomyelitis | 33/m | 0.121 | 0.365 | 0.201 |
| Myelitis | 25/f | 0.075 | 0.075 | 0.229 |
| Hereditary spastic paraplegia | 56/m | 0.011 | 0.205 | 0.637 |
| Paraneoplastic myelitis (anti-Hu antibodies positive) | 51/f | 0.005 | 0.088 | 0.017 |
| Paraneoplastic cerbellitis | 46/m | 0.0 | 0.201 | 0.204 |
| Neurosarcoidosis | 68/m | 0.0 | 0.075 | 0.184 |
| Myelitis | 54/f | 1.029 | 0.079 | 0.082 |
| Meralgia paraesthetica | 21/f | 0.025 | 0.085 | 0.176 |
| Transient ischemic attack | 71/f | 0.113 | 0.069 | 0.134 |
| Neurosarcoidosis | 35/f | 0.005 | 0.113 | 0.009 |
| Neurovasculitis (positive for anti-double stranded DNA antibody) | 59/f | 1.37 | 7.99 | 0.01672 |

### RNA Isolation and global miRNome analysis

After adding spiked-in control C. elegans miR-39 as an internal control, total RNA was isolated from 200µl CSF using the miRNeasy Mini Kit (Qiagen, Hilden, Germany), according to the manufacturer's instructions. A miRNA transcriptome analysis in pooled liquor from n=10 MS patients and n=10 patients with OND was performed by using a miRNome microRNA Profilers QuantiMir Human PCR array (Biocat, Cat.: RA660A-1, Version 15, Heidelberg, Germany) as to the manufacturers instructions and using a robotic-assisted automatic pipetting device (Agilent, Santa Clara, USA).

Detection and Quantification of miRNAs by Quantitative RT-PCR miR-181c, miR-633 and miR-922 were validated by quantitative miRNA RT-PCR technology (TaqMan MicroRNA Assays, Applied Biosystems) in n=53 MS patients and n=39 OND. Values were normalized to spiked-in cel-miR-39 and are expressed as (Starting Quantity [microRNA]/Starting Quantity [cel-miR-39]).

### Statistical analysis

Two-sided non-parametric t-test (Mann-Whitney test) was performed for all statistical analyses (GraphPad Prism, La Jolla, USA). P<0.05 (*), p<0.001 (**) and p<0.0001 (***) were considered significant. Receiver operating characteristics curves were generated for spcecificity and sensitivity values (GraphPad), logistic regression was performed for relative risks (RR) and 95% confidence intervals (95% CI) (Stata, Texas, USA).

### Example 1: miRNAs in the CSF of MS and OND patients

The candidate miRNAs identified by miRnome analyses were validated by quantitative miRNA RT-PCR, which revealed the following results: in MS CSF miR-922 (p=0.0001) was downregulated, whereas miR-181c (p=0.0007) and miR-633 (p=0.0014) were upregulated as compared to OND (**Fig. 1A-C**). None of the three candidate miRNAs correlated with age or gender. Further analyses of miRNA levels in MS patients with either relapsing remitting (RRMS, n=17) or secondary progressive (SPMS, n=30) showed significantly downregulated levels of miR-633 (p=0.0005) and miR-181c (p=0.02) in RRMS as compared to SPMS (**Fig. 1A-C**). The candidate miRNAs did not correlate with MS disease duration.

### Example 2: Diagnostic value of candidate miRNAs

In order to evaluate the diagnostic value of the candidate miRNAs we determined miRNA specific cut-off levels deduced from receiver operating characteristics curves. Combining candidate miRNAs in a diagnostic tree, as shown in figure 2A and B, resulted in enhanced specificity and sensitivity values. The combination of a cut-off value < 0.35 for miR-633 (RR, 1.9, 95% CI, 1.2 to 2.9; p=0.0065) and > 0.47 for miR-181c (RR, 2.0; 95% CI, 1.1 to 3.7; p=0.011) led to 69% sensitivity and 82% specificity for the discrimination between RRMS and SPMS **(****Fig. 2A****).** Accordingly, the combination of a cut-off value > 0.73 for miR-633 (RR, 6.1; 95% CI 2.0 to 18.2; p<0.0001) and < 0.04 for miR-922 (RR, 2.2; 95% CI, 1.4 to 3.4; p<0.0001) led to 88% sensitivity and 69% specificity for the differentiation between MS and OND (**Fig. 2B**).

The examples demonstrate that in the CSF of MS patients three distinct miRNAs are deregulated, namely miR-181c, miR-633 and miR-922 (**Fig. 1**). The MS patients included in the study had been well characterized with regard to course of disease, allowing a CSF miRNA-based discrimination according to MS disease course. Notably, miR-181c and miR-633 are differentially deregulated in RRMS and SPMS. The combination of the expression patterns found in the analyzed CSF samples by means of a diagnostic tree led to feasible diagnostic specificity and sensitivity levels (**Fig. 2A**). In addition, MS CSF revealed deregulated levels of all three miRNAs when compared to OND (**Fig. 1**). Notably, besides neurodegenerative diseases, the OND cohort tested also included patients with various neuroinflammatory disorders, such as neuromyelitis optica that are among differential diagnoses of MS (**Table 3**), which supports the potential diagnostic value of these CSF-based miRNAs (**Fig. 2B**).

## Claims

1. A method of determining if a patient is afflicted with multiple sclerosis (MS), comprising determining in a cerebrospinal fluid (CSF) sample from the patient the expression profile of miR-633 and miR-922, wherein miR-633 is up-regulated (increased concentration) and miR-922 is down-regulated (lower concentration) in a patient who is afflicted with MS.

2. The method of claim 1, further comprising, differentiating between MS and an other neurological disease (OND), wherein miR-633 is up-regulated (increased concentration) and miR-922 is down-regulated (lower concentration) in the CSF from a MS patient compared to a patient with an OND.

3. The method of claim 1 or 2, further comprising, determining the expression profile of miR-181c_5p in the cerebrospinal fluid (CSF) sample from the patient.

4. The method of claim 3, wherein miR-181c_5p is up-regulated (increased concentration) in the CSF of a patient who is afflicted with MS, or in the CSF of a MS patient compared to a patient with an OND.

5. The method of any one of claims 1 to 4, further comprising, determining the level of one or more normalization control(s) in the sample.

6. The method of claim 5, wherein the normalization control is a non-endogenous RNA or miRNA, or a miRNA not expressed in the sample.

7. The method of any one of claims 1 to 6, wherein the miRNA profile is determined by an amplification- and/or hybridization-based assay.

8. The method of claim 7, wherein the amplification- and/or hybridization-based assay is quantitative miRNA real-time polymerase chain reaction (RT-PCR).

9. The method of any one of claims 3 to 8, further comprising, discriminating between relapsing remitting multiple sclerosis (RRMS) and a progressive form of MS, wherein miR-181c_5p and miR-633 are down-regulated (lower concentration) in RRMS as compared to the progressive form of MS.

10. The method of claim 9, wherein the progressive form of MS is SPMS.

11. A kit for diagnosing or monitoring MS consisting of oligonucleotide probes or primers specifically hybridizing, reverse transcribing, or amplifying miR-633, miR-922 and miR-181c_5p.

12. Use of a miRNA expression profile of miR-633 and miR-922 in a CSF sample from a patient for diagnosing or monitoring MS.

13. The use of a miRNA profile according to claim 12, wherein the miRNA profile further comprises the level of miR-181c_5p.

14. The use of a miRNA profile according to claim 12 or 13, wherein up-regulation (increased concentration) of miR-181c_5p and/or miR-633, and down-regulation (lower concentration) of miR-922 in the sample is indicative of the presence of MS.

15. Use of a miRNA expression profile of miR-633 and/or miR-181c_5p, and miR-922 in a CSF sample from a patient for differentiating between MS and an other neurological disease (OND).

16. The use of a miRNA profile according to claim 15, wherein miR-633 and/or miR-181c_5p is/are up-regulated (increased concentration), and miR-922 is down-regulated (lower concentration) in the CSF from a MS patient compared to a patient with an OND.

17. Use of a miRNA expression profile of miR-181c_5p and miR-633 in a CSF sample from a patient for discriminating between relapsing remitting multiple sclerosis (RRMS) and a progressive form of MS, wherein miR-181c_5p and miR-633 are down-regulated (lower concentration) in RRMS as compared to the progressive form of MS.

## Patentansprüche

1. Verfahren zur Bestimmung, ob ein Patient mit Multipler Sklerose (MS) befallen ist, umfassend die Bestimmung des Expressionsprofils von miR-633 und miR-922 in einer Probe von zerebrospinaler Flüssigkeit (CSF) des Patienten, wobei miR-633 hoch reguliert ist (erhöhte Konzentration) und miR-922 nach unten reguliert (geringere Konzentration) ist in einem Patienten, der mit MS befallen ist.

2. Das Verfahren nach Anspruch 1, ferner umfassend die Differenzierung zwischen MS und einer anderen neurologischen Erkrankung (OND), wobei miR-633 hoch reguliert ist (erhöhte Konzentration) und miR-922 nach unten reguliert ist (niedrigere Konzentration) in der CSF aus einem MS-Patienten im Vergleich zu einem Patienten mit einer OND.

3. Das Verfahren nach Anspruch 1 oder 2, ferner umfassend die Bestimmung des Expressionsprofils von miR-181c_5p in der Probe von zerebrospinaler Flüssigkeit (CSF) aus dem Patienten.

4. Das Verfahren nach Anspruch 3, wobei miR-181c_5p hoch reguliert ist (erhöhte Konzentration) in der CSF eines Patienten, der mit MS befallen ist, oder in der CSF von einem MS-Patienten im Vergleich zu einem Patienten mit einer OND.

5. Das Verfahren nach einem der Ansprüche 1 bis 4, ferner umfassend die Bestimmung des Spiegels von einer oder mehreren Normalisierungskontrolle(n) in der Probe.

6. Das Verfahren nach Anspruch 5, wobei die Normalisierungskontrolle eine nicht-endogene RNA oder miRNA ist, oder eine miRNA, die in der Probe nicht exprimiert wird.

7. Das Verfahren nach einem der Ansprüche 1 bis 6, wobei das miRNA-Profil durch einen Assay bestimmt wird, der auf Amplifikation und / oder Hybridisierung basiert.

8. Das Verfahren nach Anspruch 7, wobei der Assay, der auf Amplifikation und / oder Hybridisierung basiert, quantitative miRNA Echtzeit-Polymerase-Kettenreaktion (RT-PCR) ist.

9. Das Verfahren nach einem der Ansprüche 3 bis 8, ferner umfassend die Unterscheidung zwischen schubförmig remittierender Multipler Sklerose (RRMS) und einer fortschreitenden Form von MS, wobei miR-181c_5p und miR-633 nach unten reguliert sind (geringere Konzentration) bei RRMS im Vergleich zu der fortschreitenden Form von MS.

10. Das Verfahren nach Anspruch 9, wobei die fortschreitende Form von MS SPMS ist.

11. Kit für die Diagnose oder die Überwachung von MS, bestehend aus Oligonucleotidsonden oder Primern, die spezifisch an miR-633, miR-922 und miR-181c_5p hybridisieren, miR-633, miR-922 und miR-181c_5p revers transkribieren, oder miR-633, miR-922 und miR-181c_5p amplifizieren.

12. Verwendung eines miRNA Expressionsprofils von miR-633 und miR-922 bei einer CSF-Probe von einem Patienten für die Diagnose oder Überwachung von MS.

13. Die Verwendung eines miRNA Profils nach Anspruch 12, wobei das miRNA Profil ferner den Spiegel von miR-181c_5p umfasst.

14. Die Verwendung eines miRNA Profils nach Anspruch 12 oder 13, wobei die Hochregulierung (erhöhte Konzentration) von miR-181c_5p und / oder miR-633, und eine Herunterregulierung (niedrigere Konzentration) von miR-922 in der Probe ein Hinweis auf das Vorhandensein von MS ist.

15. Verwendung eines miRNA Expressionsprofils von miR-633 und / oder miR-181c_5p, und miR-922 bei einer CSF-Probe von einem Patienten für die Differenzierung zwischen MS und einer anderen neurologischen Erkrankung (OND).

16. Die Verwendung eines miRNA Profils nach Anspruch 15, wobei miR-633 und / oder miR-181c_5p hoch reguliert (erhöhte Konzentration) ist / sind, und miR-922 nach unten reguliert (niedrigere Konzentration) ist in der CSF von einem MS-Patienten im Vergleich zu einem Patienten mit einer OND.

17. Verwendung eines miRNA Expressionsprofils von miR-181c_5p und miR-633 bei einer CSF-Probe von einem Patienten zur Unterscheidung zwischen schubförmig remittierender Multipler Sklerose (RRMS) und einer fortschreitenden Form von MS, wobei miR-181c_5p und miR-633 nach unten reguliert sind (niedrigere Konzentration) bei RRMS im Vergleich zu der fortschreitenden Form von MS.

## Revendications

1. Procédé permettant de déterminer si un patient est atteint de la sclérose en plaques (SEP), comprenant le fait de déterminer dans un échantillon de liquide céphalo-rachidien (LCR) provenant du patient le profil d'expression de miR-633 et de miR-922, où miR-633 est régulé à la hausse (concentration accrue) et miR-922 est régulé à la baisse (concentration plus faible) chez un patient qui est atteint de SEP.

2. Procédé de la revendication 1, comprenant en outre, le fait de faire la différence entre la SEP et une autre maladie neurologique (OND), où miR-633 est régulé à la hausse (concentration accrue) et miR-922 est régulé à la baisse (concentration plus faible) dans le LCR provenant d'un patient atteint de SEP par rapport à un patient avec une OND.

3. Procédé de la revendication 1 ou 2, comprenant en outre, le fait de déterminer le profil d'expression de miR-181c_5p dans l'échantillon de liquide céphalo-rachidien (LCR) provenant du patient.

4. Procédé de la revendication 3, dans lequel miR-181c_5p est régulé à la hausse (concentration accrue) dans le LCR d'un patient qui est atteint de SEP, ou dans le LCR d'un patient atteint de SEP par rapport à un patient avec une OND.

5. Procédé de l'une quelconque des revendications 1 à 4, comprenant en outre, le fait de déterminer le niveau d'un ou de plusieurs témoin(s) de normalisation dans l'échantillon.

6. Procédé de la revendication 5, dans lequel le témoin de normalisation est un miARN ou un ARN non-endogène, ou un miARN non exprimé dans l'échantillon.

7. Procédé de l'une quelconque des revendications 1 à 6, dans lequel le profil de miARN est déterminé par un dosage basé sur l'amplification et/ou sur l'hybridation.

8. Procédé de la revendication 7, dans lequel le dosage basé sur l'amplification et/ou sur l'hybridation est une réaction en chaîne par polymérase en temps réel de miARN quantitative (RT-PCR).

9. Procédé de l'une quelconque des revendications 3 à 8, comprenant en outre, le fait de faire une distinction entre la sclérose en plaques récurrente rémittente (SEPRR) et une forme progressive de SEP, où miR-181c_5p et miR-633 sont régulés à la baisse (concentration plus faible) dans la SEPRR par rapport à la forme progressive de la SEP.

10. Procédé de la revendication 9, dans lequel la forme progressive de la SEP est la SEPPS.

11. Kit pour diagnostiquer ou surveiller la SEP constitué d'amorces ou de sondes oligonucléotidiques s'hybridant spécifiquement à miR-633, miR-922 et miR-181c_5p, effectuant une transcription inverse de ceux-ci, ou les amplifiant.

12. Utilisation d'un profil d'expression de miARN de miR-633 et de miR-922 dans un échantillon de LCR provenant d'un patient pour diagnostiquer ou surveiller la SEP.

13. Utilisation d'un profil de miARN selon la revendication 12, dans laquelle le profil de miARN comprend en outre le niveau de miR-181c_5p.

14. Utilisation d'un profil de miARN selon la revendication 12 ou 13, dans laquelle la régulation à la hausse (concentration accrue) de miR-181c_5p et/ou de miR-633, et la régulation à la baisse (concentration plus faible) de miR-922 dans l'échantillon indiquent la présence de la SEP.

15. Utilisation d'un profil d'expression de miARN de miR-633 et/ou de miR-181c_5p, et de miR-922 dans un échantillon de LCR provenant d'un patient pour faire la différence entre la SEP et une autre maladie neurologique (OND).

16. Utilisation d'un profil de miARN selon la revendication 15, dans laquelle miR-633 et/ou miR-181c_5p est/sont régulé(s) à la hausse (concentration accrue), et miR-922 est régulé à la baisse (concentration plus faible) dans le LCR provenant d'un patient atteint de SEP par rapport à un patient avec une OND.

17. Utilisation d'un profil d'expression de miARN de miR-181c_5p et de miR-633 dans un échantillon de LCR provenant d'un patient pour faire la distinction entre la sclérose en plaques récurrente rémittente (SEPRR) et une forme progressive de la SEP, où miR-181c_5p et miR-633 sont régulés à la baisse (concentration plus faible) dans la SEPRR par rapport à la forme progressive de la SEP.
